# EUROPEAN PATENT SPECIFICATION

(11) **EP 1 608 678 B1**
(45) Date of publication and mention of the grant of the patent: **22.08.2007**
(21) Application number: 04723460.4
(22) Date of filing: 26.03.2004
(51) Int. Cl.: C07K 14/435, A23J 1/06, B01D 15/00

(54) **PROCESS FOR ISOLATING HAEMOCYANIN**
VERFAHREN ZUR ISOLIERUNG VON HÄMOCYANIN
PROCEDE POUR ISOLER L'HEMOCYANINE

(30) Priority: 28.03.2003 AU 2003901507 P
(43) Date of publication of application: 28.12.2005
(73) Proprietor: Norika Holdings Pty Ltd, Kedron, QLD 4031 (AU)
(72) Inventor: MANICKAVASAGAM, Bhanu, Chapel Hill, Queensland 4069 (AU)
(74) Representative: Banford, Paul Clifford
(86) International application number: PCT/AU2004/000382
(87) International publication number: WO 2004/085467

(56) References cited:
- WO-A-99/62936
- WO-A1-02/102844
- LIPNIZKI F ET AL: "Concepts of industrial-scale diafiltration systems" DESALINATION, ELSEVIER, AMSTERDAM, NL, vol. 144, no. 1-3, 10 September 2002 (2002-09-10), pages 179-184, XP004386216 ISSN: 0011-9164
- SWERDLOW R D ET AL: "KEYHOLE LIMPET HEMOCYANIN: STRUCTURAL AND FUNCTIONAL CHARACTERIZATION OF TWO DIFFERENT SUBUNITS AND MULTIMERS" COMPARATIVE BIOCHEMISTRY AND PHYSIOLOGY. B. COMPARATIVE BIOCHEMISTRY, PERGAMON PRESS, LONDON, GB, vol. 113, 1996, pages 537-548, XP000900921 ISSN: 0305-0491
- SCHUTZ J. ET AL.: 'Isolation and spectroscopic characterization of the structural subunits of Keyhole Limpet Hemocyanin' BIOCHIMICA ET BIOPHYSICA ACTA vol. 1546, no. 2, 07 April 2001, pages 325 - 336, XP004279187
- MILLER K.I. ET AL.: 'Structure and function of the carboxyl-terminal oxygen-binding domain from the subunit of Octopus dofleini Hemocyanin' BIOCHEMISTRY vol. 27, 1988, pages 7282 - 7288, XP002999241
- SULLIVAN B. ET AL.: 'Hemocyanin of the Horseshoe Crab, Limulus polyphemus' THE JOURNAL OF BIOLOGICAL CHEMISTRY vol. 251, no. 23, 1976, pages 7644 - 7648, XP002999242

## Description

### Technical Field

The present invention is concerned with a process for isolating a pharmaceutical product, specifically a serum protein identified as haemocyanin, from the blood of abalone.

### Background of the Invention

Abalone has been found to be the source of a number of useful materials which serve as replacements for similar materials from other sources or are novel products, as described in International Publication No. WO 02/102851.

Haemocyanin (Hc) is the blue, copper-containing respiratory protein of many molluscs and arthropods, Haemocyanins are always found freely dissolved in the blood (or hemolymph). The molluscan haemocyanins have an entirely different structure and arrangement of subunits compared to arthropod haemocyanins. Aerobic metabolism of molluscs such as abalone is supported by gas exchange through gills found in the respiratory cavity. The blood pumped through the gills, via a low-pressure open circulatory system, contains haemocyanin which transports oxygen to respiratory tissues. In open systems blood flows from arteries into the tissue spaces and finally into venous sinuses before being collected in veins and returned to the heart. Oxygenated blood ranges from pale to strong blue depending on the degree of oxygenation, haemocyanin concentration and species of animal. Dimeric copper pairs in the haemocyanin provide reversible sites for the binding of one oxygen molecule. Haemocyanin is also a source of copper that may lead to inorganic and organic blueing reactions in abalone food processing.

Haemocyanins are arranged into multi-subunit proteins which carry as few as six or as many as several hundred oxygen molecules. Molluscan haemocyanins are extremely large macromolecules having molecular masses of around 4 million daltons (Da). Molluscan haemocyanins have subunits containing seven or eight oxygen binding functional units. Each globular functional unit is of about 50 kDa and they are arranged like a string of beads. Ten such subunits assemble to form cylindrical decameric whole molecules, and in gastropods multiples of two or more decamers may be found. The wall of the decamer has sixty oxygen binding units, and the remaining units form the so-called collar which lies in the centre of the cylinder and, in the case of gastropod haemocyanin, offset to one end. The association of haemocyanin subunits requires divalent cations, either Mg²⁺ or Ca²⁺, as well as competent monomers (Mangum, 1983). Abalone haemocyanin have a similar structure to other molluscan haemocyanin but it has been reported that they differ from other haemocyanins (and from haemoglobulin) in that oxygen binding has an allosteric mechanism (Behrens et al., 2002) dependent on both Mg²⁺ and Ca²⁺.

The copper content of molluscan haemocyanins averages around 0.25%, corresponding to 1 gram atom per 25000 daltons of protein. Haemocyanins are potent immunogens which induce the synthesis of large amounts of specific antibodies. The Hc may exist in associated or dissociated forms (Bartell and Campbell, 1959). In addition to containing associated or dissociated Hc molecules, various preparations may contain a number of other immunologically distinct proteins. For instance the hemolymph of the crab may contain at least 5 distinct proteins as well as two electrophoretically distinct Hc (Horn and Kerr, 1969), as do the haemocyanins from abalone, keyhole limpet haemocyanin and octopus at least (Miller *et al.,* 1998).

International Publication No. WO 02/102844 describes a laboratory scale process for the isolation of haemocyanin from abalone in which a phenyl hydrophobic interaction chromatography (phenyl HIC) column is used to separate haemocyanin from a haemocyanin-containing solution. Hydrophobic interaction chromatography is a separation procedure based on the attraction between hydrophobic groups on the protein and a hydrophobic ligand. In this instance sufficient interaction was found between the phenyl group of the phenyl HIC resin and haemocyanin for the haemocyanin to be retained on the column in laboratory scale experiments, but this process has been found to be unsuitable for scaling up to a commercially viable level of production. Further laboratory scale attempts to isolate haemocyanin are described by Harris *et al.* and Lieb *et al.* The Harris paper describes the isolation of haemocyanin from *Haliotis tuberculata* through the use of anion exchange chromatography using Q-Sepharose gel and an elution buffer with pH 7.4. Lieb *et al*. also describe the isolation of haemocyanin from *H*. *tuberculata* haemolymph using anion exchange chromatography, this time with a Mono-Q column with the elution performed using a sodium chloride step gradient. Both papers are concerned with the nature of the haemocyanin molecule, and only sufficient material to undertake an analysis of the molecule has been isolated only. Accordingly, there remains a need for a commercial scale preparation of this product.

### Summary of the Invention

According to a first aspect of the present invention there is provided a process for obtaining a stable solution of a molluscan haemocyanin, comprising the steps of:
(1) collecting blood from a mollusc;
(2) centrifuging the collected blood to remove cellular material and other particulates to obtain a serum fraction;
(3) diafiltering the serum fraction to replace serum liquid with a cation exchange equilibration buffer using an ultrafiltration membrane;
(4) contacting the serum fraction with a resin in the presence of an equilibration buffer with a pH between 4.0 and 6.5, whereby a protein component of the serum fraction is retained on the column;
(5) eluting the retained protein component from the column with a high salt elution buffer; and
(6) concentrating the eluate and exchanging the elution buffer for a pH neutral/low salt diafiltration buffer by recirculating the eluate through an ultrafiltration membrane in the presence of the diafiltration buffer,
characterised in that the resin is a sulphonic acid-containing strong cation exchange resin and the sulphonate groups are immobilised to methacrylate copolymer beads.

The strong cation exchange resin is formed as a column packed with a sulfonic acid-containing resin such as the Bio-Rad Macro Prep High S resin, in which sulfonate groups are attached to a solid support, in this instance methacrylate coplymer beads.

Advantageously the elution buffer comprises a weak acid, preferably an organic acid such as acetic acid, propionic acid or butyric acid, most preferably acetic acid.

The elution buffer preferably has a pH between 4.0 and 6.5, more preferably between 5.0 and 6.0 and most preferably 5.5. It will be appreciated that the optium pH for retention of haemocyanins from different molluscs may vary within this range dependent upon the charge profile of the protein. Hence, the selection of the pH of the buffer and buffer composition will depend upon the source of the haemocyanin as would be understood by the person skilled in the art. At a pH substantially below 4.0 it may be expected that molluscan haemocyanin will precipitate on the column and so the process will not be effective. At a pH substantially above 6.5 the degree of ionisation of the protein is likely to be unsuitable for retention on a cation exchange resin.

In a particularly preferred embodiment of the invention the elution buffer comprises acetic acid, magnesium chloride and calcium chloride at pH 5,5.

Typically the high salt elution buffer is weakly acidic, and preferably has the same composition as the equilibration buffer with the exception that sodium chloride is added thereto. In particular, the elution buffer may have the same composition as the equilibration buffer provided that it also contains sodium chloride, typically IM sodium chloride. Nevertheless, it will be recognised by the person skilled in the art that other elution buffers may be devised which are suitable, and any such buffer is envisaged.

Typically the diafiltration buffer is suitable for long term storage of the serum protein. It is relatively pH neutral and has a low salt concentration, and typically comprises Na₂HPO₄ + NaH₂PO₄ + sodium chloride at pH 7.2, but the person skilled in the art will be able to devise other suitable buffers.

Advantageously the ultrafiltration membrane has a molecular weight cut-off of 100KD.

The serum protein isolated in the process of the present invention has been identified as haemocyanin. It has been found that use of a strong cation exchange resin at a weakly acidic pH is suitable for the isolation of molluscan haemocyanin in a process which is scaleable with good yield, and in which the haemocyanin is not degraded to any marked extent.

There is also provided a haemocyanin when isolated by the process of the first aspect.

Advantageously, the mollusc is selected from the group consisting of abalone, octopus and limpet, preferably abalone and more preferably one of the commercial species such as the black-lip abalone, *Haliotis* rube, the brown-lip abalone, *Haliotis conicopora,* the green-lip abalone, *Haliotis laevigita,* and Roe's abalone, *Haliotis roei.*

### Brief Description of the Drawings

Preferred embodiments of the present invention will now be described, by way of example only, with reference to the accompanying figures, in which:
Fig. 1 is a graph of absorbance versus cumulative volume and shows elution of the serum protein of the invention; and
Fig. 2 is a SDS-PAGE gel of the sterile filtered final product.

### Modes for Performing the Invention

### Example 1

The novel process of the invention, in one embodiment, is performed in a pilot plant equipped with a tangential flow filtration device (Millipore Prep Scale ™ TFF-6) with a filter area of 0.557418 square meters [6 square feet]. The process comprises the following steps:
Step 1. Whole live abalone are obtained and may be processed immediately or stored in an appropriate tank until required.
Step 2. Rinse abalone under running water prior to shucking. Working on a chopping board, shuck the animal and remove the body from the shell.
Step 3. Carefully cut around the top of the foot to remove the guts.
Step 4. Rinse the foot under running water prior to bleeding. Working in a clean container to collect any blood, cut away the mouth area and store for later use. Make several deep incisions across the front of the foot. Store the initial blood collection in a coldroom.
Step 5. Quickly transfer the foot to a draining tray above a collection vessel. Stand the abalone upright in the tray and cover. Allow the blood to drain overnight in a coldroom.
Step 6. Remove any solid material from the blood by centrifugation at 12000 x g. Store the supernatant in a coldroom until required.
Step 7. Prepare 12 column volumes + 8 supernatant volumes of equilibration buffer. The equilibration buffer consists of 18 mM acetic acid + 1 mM MgCl₂ + 1 mM CaCl₂ at pH 5.5.
Step 8. Prepare 5 column volumes of elution buffer per column run. The elution buffer consists of 18 mM acetic acid + 1 M NaCl + 1 mM MgCl₂ + 1 mM CaCl₂ at pH 5.5.
Step 9. Pack a suitably sized column (Pharmacia Index 140-500) with 5L of Bio-Rad Macro-Prep High S resin and equilibrate with equilibration buffer for at least 7 column volumes and until the pH and conductivity of the column outflow are within 0.05 pH units and 0.5 mS of the buffer. The maximum running flow rate was 500ml/min.
Step 10. Buffer exchange of the supernatant against, the equilibrium buffer is performed with a 100 kD NMWCO ultrafiltration cartridge.
Step 11. Diafilter the supernatant for at least 6 supernatant volumes and until the supernatant conductivity is within 0.5 mS of the equilibrium buffer. Collect the diafiltration permeate.
Step 12. Concentrate the diafiltered supernatant down to 3 system hold-up volumes less than the original supernatant volume. Drain the system hold-up and add to the retentate. Rinse the cartridge by recirculating 1.5 hold-up volumes of equilibration buffer at the operating flowrate for 10 minutes. Drain the system hold-up and add to the retentate.
Step 13. Prepare 2L of ultrafiltration cartridge cleaning solution per m² of membrane area. The storage solution consists of 1 M NaOH at 40°C. Clean the cartridge by recirculating the cleaning solution for at least 30 minutes. Rinse the cartridge with deionised water until the pH of the retentate and permeate are < 7.
Step 14. Prepare 2L of ultrafiltration cartridge storage solution per m² of membrane area. The storage solution consists of 0.1 M NaOH or 20% ethanol. Rinse the cartridge with storage solution then seal and store the cartridge in a coldroom.
Step 15. Begin loading the column at the running flowrate. Measure the absorbance at 280 nm of the column outflow and collect the UV absorbing flow through fractions. Plot the UV absorbance of the fractions against the cumulative volume collected.
Step 16. Wash the column with at least 3 column volumes of equilibration buffer and until the absorbance at 280 nm of the column outflow has reached baseline. Continue to collect the UV absorbing flow through fractions and plot on the chromatogram.
Step 17. Elute the column with at least 3 column volumes of elution buffer and until the absorbance at 280 nm of the column outflow has reached baseline. Collect the UV absorbing elution fractions and plot on the chromatogram.
Step 18. Repeat from the equilibrium step (step 9) for up to 2 additional column runs. Further runs will require column cleaning (steps 19 and 20) every 3 runs.
Step 19. Prepare 2 column volumes of cleaning in place solution. The CIP solution consists of 1 M NaOH. Clean the column at the running pressure. Collect the UV absorbing CIP fractions and plot on the chromatogram.
Step 20. Wash the column with at least 2 column volumes of equilibration buffer and until the absorbance at 280 nm of the column outflow has reached baseline and the pH of the column outflow < 7. Continue to collect the UV absorbing fractions and plot on the chromatogram.
Step 21. Prepare 1 column volume of storage solution. The storage solution consists of 20% ethanol. Rinse the column at the running flowrate. Seal, label, and store the packed column.
Step 22. Conduct a protein assay on the chromatography samples and pool the elutions. Calculate the volume required for a 20 mg/ml solution of final product.
Step 23. Prepare 8 product volumes of diafiltration buffer. The diafiltration buffer consists of 53 mM Na₂HPO₄ + 30 mM NaH₂PO₄ + 150 mM NaCl at pH 7.2.
Step 24. Final concentration and buffer exchange of the product is performed with a 100 kD NMWCO ultrafiltration cartridge.
Step 25. Concentrate the pooled column elutions down to the volume calculated in step 22. Collect the ultrafiltration permeate.
Step 26. Diafilter the ultrafiltration retentate for at least 6 retentate volumes and until the retentate pH is within 0.03 pH units of the diafiltration buffer. Collect the diafiltration permeate.
Step 27. Concentrate the diafiltered product down to 3 system hold-up volumes less than the required final product volume. Drain the system hold-up and add to the retentate. Rinse the cartridge by recirculating 1.5 hold-up volumes of diafiltration buffer at the operating flowrate for 10 minutes. Drain the system hold-up and add to the retentate.
Step 28. Prepare 2 L of ultrafiltration cartridge cleaning solution per. m² of membrane area. The cleaning solution consists of 1 M NaOH at 40°C. Clean the cartridge by recirculating the cleaning solution for at least 30 minutes. Rinse the cartridge with deionised water until the pH of the retentate and permeate are < 7.
Step 29. Prepare 2 L of ultrafiltration cartridge storage solution per m² of membrane area. The storage solution consists of 0.1 M NaOH or 20% ethanol. Rinse the cartridge with storage solution then seal and store the cartridge in a coldroom.
Step 30. Conduct a protein assay on the ultrafiltration samples. Recalculate the volume required for a 20 mg/ml solution of final product. Make up the retentate volume with diafiltration buffer or repeat steps 25-27 as required.
Step 31. Prepare a glove box by turning on the UV light and filter for at least 30 minutes. Working in the glove box, sterile filter the final product through a 0.2 pm filter into a sterile container(s).
Step 32. Conduct QA analysis of the final product.

### Results

The pilot scale process was successful in producing a high quality product. Approximately 8L of blood was processed to produce over a litre of final product in a period of nine days.

Pre-treatment studies showed no loss of protein with either centrifugation or filtration, indicating the feasibility of either method. Centrifugation may be the efficient option considering the low solids content and high viscosity of the blood.

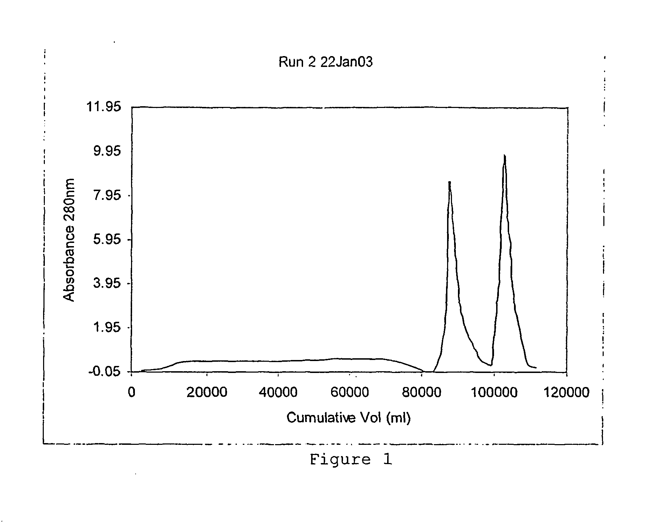

The column purification step exhibited good flow characteristics and thus reasonable run times. The chromatogram for the second chromatography run is shown in Fig. 1. The broad, flat, first peak is the flow through of unbound protein, the sharp, second peak is the salt elution of bound protein, and the slightly taller last peak is the protein removed by the cleaning step.

The binding of protein over the two chromatography runs was 61%. This could be improved by reducing the volume of blood loaded onto the column. The yield of bound protein recovered by the salt elution was around 88%. This could be improved by increasing the salt concentration.

Concentration and buffer exchange of the protein by ultrafiltration was very efficient, taking around 5 hours with no protein losses to the permeate.

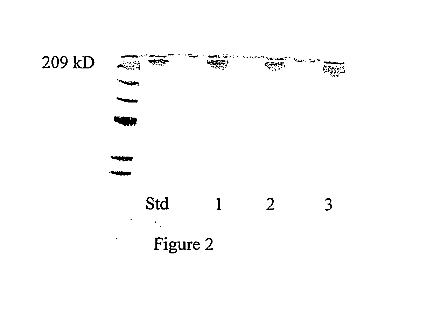

The SDS-PAGE analysis of the sterile filtered final product is shown in the gel of Fig. 2. The three filtration batches are designated 1, 2, 3 and can be seen to be similar to the standard in molecular weight and single band purity.

Streaking of the undiluted final filtration batches onto agar plates produced no microbial growth from any of the samples, indicating that sterility has been achieved.

Throughout this specification and the claims, the words "comprise", "comprises" and "comprising" are used in a non-exclusive sense, except where the context requires otherwise.

It will be clearly understood that, although a number of prior art publications are referred to herein, this reference does not constitute an admission that any of these documents forms part of the common general knowledge in the art, in Australia or in any other country.

### Industrial Applicability

The process of the present invention provides a commercial scale process for the isolation of haemocyanin from the blood of molluscs such as abalone. The haemocyanin is useful as a pharmaceutical agent, particularly as an anti-tumour agent, especially for bladder cancer. It is also useful as an ingredient in cosmetic formulations. In addition, it is useful as an immunoadjuvant. It may also be used as a laboratory tool in the life sciences, for example to coat ELISA plates, and in chromatography media.

### References

Bartell, A.H. and Campbell D.H. (1959) Arch. Biochem. Biophys. 82, 232.
Behrens et al., J. Exp. Biol. 205, 253-263 (2002).
Harris, J.O., Maguire, G.B., Edwards, S. and Hindrum, S.M. (1998). Aquaculture 160, 3-4, 259.
Horn B.C and Kerr (1969). Comp. Biochem. Physiol. 29, 493.
Lieb et al., Eur. J. Biochem, 265, 134-144 (1999).
Mangum, C.P., (1983). Oxygen Transport in the blood. In: Mantel, L.H. (Ed.); Bliss, D.E. (Series Ed.), The Biology of Crustacea. Vol. 5. Internal Anatomy and Physiological Regulation. Academic Press, New York, pp. 373-429.
Miller at al., J. Mol. Biol. 278, 827-842 (1998).

## Claims

1. A process for obtaining a stable solution of a molluscan haemocyanin, comprising the steps of:
(1) collecting blood from a mollusc;
(2) centrifuging the collected blood to remove cellular material and other particulates to obtain a serum fraction;
(3) diafiltering the serum fraction to replace serum liquid with a cation exchange equilibration buffer using an ultrafiltration membrane;
(4) contacting the serum fraction with a resin in the presence of an equilibration buffer with a pH between 4.0 and 6.5, whereby a protein component of the serum fraction is retained on the column;
(5) eluting the retained protein component from the column with a high salt elution buffer; and
(6) concentrating the eluate and exchanging the elution buffer for a pH neutral/low salt diafiltration buffer by recirculating the eluate through an ultrafiltration membrane in the presence of the diafiltration buffer, **characterised in that** the resin is a sulphonic acid containing strong cation exchange resin and the sulfonate groups are immobilised to methacrylate copolymer beads.

2. A process as claimed in claim 1 wherein the pH of the equilibration buffer is between 5.0 and 6.0.

3. A process as claimed in claim 2 wherein the pH of the equilibration buffer is 5.5.

4. A process as claimed in any one of claims 1 to 3 wherein the elution buffer comprises an organic acid.

5. A process as claimed in Claim 4 wherein the organic acid is selected from the group consisting of acetic acid, propionic acid and butyric acid.

6. A process as claimed in claim 5 wherein the elution buffer further comprises magnesium chloride and calcium chloride.

7. A process as claimed in any one of claims 1 to 6 wherein the elution buffer contains sodium chloride.

8. A process as claimed in claim 7 wherein the elution buffer contains 1M sodium chloride.

9. A process as claimed in any one of claims 1 to 8 wherein the the mollusc is selected from the group consisting of abalone, octopus and limpet.

10. A process as claimed in claim 9 wherein the mollusc is abalone.

11. A process as claimed in claim 10 wherein the abalone is selected from the group consisting of the black-lip abalone, *Haliotis ruber*, the brown-lip abalone, *Haliotis conicopora,* the green-lip abalone, *Haliotis laevigita,* and Roe's abalone, *Haliotis roei*.

## Patentansprüche

1. Verfahren zur Gewinnung einer stabilen Lösung eines Mollusken-Hämocyanins mit den folgenden Schritten:
(1) Sammeln von Blut von einer Molluske;
(2) Zentrifugieren des gesammelten Bluts, um Zellmaterial und andere Schwebeteilchen zu entfernen und eine Serumfraktion zu gewinnen.
(3) Diafiltration der Serumfraktion, um mit Hilfe einer Ultrafiltrationsmembran Serumflüssigkeit durch einen Kationcnaustausch-Äquilibricrungspuffer zu ersetzen:
(4) Inkontaktbringen der Serumfraktion mit einem Harz in Gegenwart eines Äquilibrierungspuffers mit einem pH-Wert zwischen 4,0 und 6,5, wodurch eine Proteinkomponente der Serumfraktion auf der Säule zurückgehalten wird;
(5) Eluieren der zurückgehaltenen Proteinkomponente aus der Säule mit einem stark salzhaltigen Elutionspuffer; und
(6) Einengen des Eluats und Austausch des Elutionspuffers gegen einen pH-neutralen/wenig salzhaltigen Diafiltrationspuffer durch Zurückführen des Eluats durch eine Ultrafiltrationsmembran in Gegenwart des Diafiltrationspuffers,
**dadurch gekennzeichnet, dass** das Harz ein sulfonsäurehaltiges starkes Kationenaustauscherharz ist und die Sulfonatgruppen an Methacrylat-Copolymerperlen immobilisiert sind,

2. Verfahren nach Anspruch 1, wobei der pH-Wert des Äquilibrierungspuffers zwischen 5,0 und 6,0 liegt.

3. Verfahren nach Anspruch 2. wobei der pH-Wert des Äquilibrierungspuffers 5,5 beträgt.

4. Verfahren nach einem der Ansprüche 1 bis 3. wobei der Elutionspuffer eine organische Säure aufweist.

5. Verfahren nach Anspruch 4, wobei die organische Säure aus der Gruppe ausgewählt ist, die aus Essigsäure, Propionsäure und Buttersäure besteht.

6. Verfahren nach Anspruch 5, wobei der Elutionspuffer ferner Magnesiumchlorid und Calciumchlorid aufweist.

7. Verfahren nach einem der Ansprüche 1 bis 6, wobei der Elutionspuffer Natriumchlorid enthält.

8. Verfahren nach Anspruch 7, wobei der Elutionspuffer 1M Natriumchlorid enthält.

9. Verfahren nach einem der Ansprüche 1 bis 8, wobei die Molluske aus der Gruppe ausgewählt ist, die aus Abalone (Seeohr), Oktopus und Napfschnecke besteht.

10. Verfahren nach Anspruch 9, wobei die Molluske Abalone ist,

11. Verfahren nach Anspruch 10, wobei die Abalone aus der Gruppe ausgewählt ist, die aus der schwarzlippigen Abalone, Haliotis ruber, der braunlippigen Abalone, Haliotis conicopora, der grünlippigen Abalone, Haliotis laevigita und der Roc-Abalone, Haliotis roei, besteht.

## Revendications

1. Procédé d'obtention d'une solution stable d'une haemocyanine de mollusque, comprenant les étapes de:
(1) recueil du sang d'un mollusque;
(2) centrifugation du sang recueilli pour retirer les matières cellulaires et les autres matières sous forme de particules pour obtenir une fraction de sérum;
(3) diafiltration de la fraction de sérum pour remplacer le liquide sérique par un tampon d'équilibrage à échange de cations en utilisant une membrane d'ultrafiltration;
(4) mise en contact de la fraction de sérum avec une résine en présence d'un tampon d'équilibrage avec un pH compris entre 4,0 et 6,5, moyennant quoi un composant protéique de la fraction de sérum est retenu sur la colonne;
(5) élution du composant protéique retenu de la colonne avec un tampon d'élution à teneur élevée en sel; et
(6) concentration de l'éluat et échange du tampon d'élution contre un tampon de diafiltration de pH neutre/faible teneur en sel par recirculation de l'éluat à travers une membrane d'ulttafiltration en présence du tampon de diafiltration, **caractérisée en ce que** la résine est une forte résine échangeuse de cations contenant un acide sulfonique et les groupes sulfonates sont immobilisés sur des billes en copolymère de méthacrylate.

2. Procédé tel que revendiqué dans la revendication 1, dans lequel le pH du tampon d'équilibrage est compris entre 5,0 et 6,0.

3. Procédé tel que revendiqué dans la revendication 2, dans lequel le pH du tampon d'équilibrage est de 5,5.

4. Procédé tel que revendiqué dans l'une quelconque des revendications 1 à 3, dans lequel le tampon d'élution comprend un acide organique.

5. Procédé tel que revendiqué dans la revendication 4, dans lequel l'acide organique est choisi parmi le groupe constitué de l'acide acétique, de l'acide propionique et de l'acide butyrique.

6. Procédé tel que revendiqué dans la revendication 5, dans lequel le tampon d'élution comprend en outre du chlorure de magnésium et du chlorure de calcium.

7. Procédé tel que revendiqué dans l'une quelconque des revendications 1 à 6, dans lequel le tampon d'élution contient du chlorure de sodium.

8. Procédé tel que revendiqué dans la revendication 7, dans lequel le tampon d'élution contient du chlorure de sodium IM.

9. Procédé tel que revendiqué dans l'une quelconque des revendications 1 à 8, dans lequel le mollusque est choisi parmi le groupe constitué de l'abalone, du poulpe et de la patelle.

10. Procédé tel que revendiqué dans la revendication 9, dans lequel le mollusque est une abalone.

11. Procédé tel que revendiqué dans la revendication 10, dans lequel l'abalone est choisie parmi le groupe constitué de l'abalone à lèvres noires, Haliotis ruber, de l'abalone à lèvres marrons, Haliotis conicopora, de l'abalone à lèvres vertes, Haliotis laevigita, et de l'abalone de Roc, Haliotis roei.
